# EUROPEAN PATENT APPLICATION

(11) **EP 1 331 479 A1**
(43) Date of publication of application: **30.07.2003**
(21) Application number: 01974666.8
(22) Date of filing: 03.10.2001
(51) Int. Cl.: G01N 33/15, G01N 33/48, G01N 33/50, G01N 33/60, A61K 51/04

(54) **TWO-DIMENSIONALLY QUANTIFIED IMAGE METHOD FOR DISTINGUISHING AND QUANTIFYING OVERGROWING TISSUE OR THE LIKE**

(30) Priority: 06.10.2000 JP 2000308513
(71) Applicant: Institute of Whole Body Metabolism, Shiroi-shi, Chiba 270-1407 (JP)
(72) Inventor: SHIGEMATSU, Akiyo, Shiroi-shi, Chiba 270-1436 (JP); HATORI, Akiko, Funabashi-shi, Chiba 273-0003 (JP); AWAZU, Shoji, Kawaguchi-shi, Saitama 333-0847 (JP)
(74) Representative: HOFFMANN - EITLE
(86) International application number: JP0108707
(87) International publication number: WO02031492

(57) **Abstract**

The present invention provides a rapid screening method for new drug candidates by providing an early visual indication of pharmacological effects [growth suppression or inhibition] in vivo of drugs administered to animals harboring an excessive growth of tissues or cells, such as malignant tumors, and the like, and a rapid method for establishing an appropriate use of therapeutic drugs.

The present invention is a two-dimensional quantitative imaging method for identifying and quantifying abnormally growing tissues or cells from normally grown tissue or cells by a two-dimensional image, preferably by a two-dimensional image analysis of a macro-autoradiograph, based on the marker [2-¹⁴C] thymidine or [1,3-N] fluorescent thymidine. Also, the present invention is a two-dimensional quantitative imaging method for simultaneously acquiring two-dimensional quantitative images of a radioactive nuclide and said thymidine. Further, the present invention, based on the aforementioned two-dimensional quantitative imaging method, is a rapid screening method for new drug candidates; a rapid method for establishing an appropriate use of therapeutic drugs; a method for determining the optimum dose of high-energy particles; a method for determining the ratio of the moiety retaining chemical stability of a therapeutic drug; a method for determining the lethal effect of high-energy particles; a method for determining the efficacy of a drug exhibiting tissue-specific efficacy; and a method for establishing an applicable dose of ⁹⁰Y.

## Description

### Field of Technology

The present invention relates to a rapid method for screening new drug candidates in living organisms where mammalian animals are used and to a rapid method for the establishment of an appropriate use of therapeutic drugs. In detail, it further relates to a rapid screening method for new drug candidates by providing an early visual indication of pharmacological effects [growth suppression or inhibition] in vivo of drugs administered to animals harboring an excessive growth of tissues or cells, such as malignant tumors, and the like; a rapid method for establishing an appropriate formula to use of therapeutic drugs; a method for judging the optimum dosage for high-energy particles; a method for determining the ratio of the moiety retaining chemical stability of a therapeutic drug; a method for determining the lethal effects of high-energy particles; a method for determining the efficacy of drugs with tissue-specific efficacy; and a method for establishing an applicable dose of ⁹⁰Y, and the like.

### Background Technology

It has always been desirable to develop drugs that are highly effective in suppressing or inhibiting the growth of abnormal tissues or cells such as malignant tumors and the like and subsequent metastases and to establish an appropriate use of such therapeutic drugs. However, many existing anti-cancer drugs not only suppress the growth of malignant cells, but also inhibit the regeneration of normal tissue. Therefore, such existing anticancer drugs cause the patients to suffer from severe side effects and may lead to premature deaths.

One treatment strategy in cancer is chemotherapy, in which drugs with anti-tumor activity are used. Most of these drugs are inhibitors or antagonists of substrates involved in nucleic acid synthesis. Recently, efforts are being made in the biotechnology field to develop antibody proteins (monoclonal antibodies) targeting enzymes (the synthetic process of DNA→RNA replication→mRNA→protein) involved in nucleic acid synthesis. However, these antibody proteins provide only slight selectivity in identifying abnormal tissues from normal tissues and have weak toxicity suppression effects (cell death). Therefore, only antibodies directed against CD20, CD30, and the like, involved in immunity, and indicated for hematologic malignancies, have been approved by the US FDA (for example Rituxan).

The use of radioactive compounds for in-vivo diagnostic drugs is a part of the mainstream in Japan and worldwide. For in vitro diagnostic agents, ¹²⁵I has been used to measure hormone levels in the blood and other components present at minute levels in the body. ⁵⁷Fe, ⁵¹Cr, ^{99m}Tc ¹³¹I, ^{115m}In, ⁷²Ga, and the like have been used as in vivo diagnostic agents. Only a limited number of radioactive nuclides have been used for therapeutic purposes, such as ¹⁹⁸Au, ⁸⁹Sr, ¹³¹I, ⁶⁰Co (external beam irradiation) and the like. (β radiation sources have been used to achieve local radiation by implanting needles embedded with the radioactive source in the body.

However, these conventional treatment methods and radiopharmaceuticals have difficulty in identifying normal from abnormal tissues or cells, have failed either to complete rapid screenings for new drug candidates or to establish the appropriate uses for a therapeutic drug in a rapid manner.

In order to distinguish abnormal from normal tissues and cells, both the abnormal and normal tissues and cells are needed to be specified in terms of the frequency of cell division and their location within the body. If a normal site has received lethal effects of a drug, it is important to determine whether or not that site can be regenerated by a procedure such as transplantation. For this purpose, attempts have been made to obtain schematic information using mammalian animal models, but no satisfactory information is yet available.

In order to complete the screening for new drug candidates or to establish the appropriate use for a therapeutic drug, it is necessary to develop a labeled indicator compound that participates in a specific period for a short time during the course of the DNA replication of abnormal tissue or cells and then ceases to participate thereafter. Compounds are known that are active only in the S phase (DNA synthesis period) during the cell cycle, of which thymidine is a well-known example. However, since it is difficult to identify the thymidine that is newly incorporated into the tissue or cell, it has been unknown when the thymidine is most active in the tissue or cells and what is the fate of the majority of the unincorporated thymidine.

The conventional method to distinguish between abnormally growing tissues or cells from normally growing tissues or cells has been to identify cells in the S phase in cultured cells or by histologic methods. For example, labeled thymidines such as [6-³H] thymidine, [2-¹⁴C] thymidine, (methyl-¹⁴C) thymidine, [methyl-1',2'-³H] thymidine, [5'-³H] thymidine, [methyl-³H] thymidine and the like, have been used. However, visualization of tissues and cells in abnormally growing or normally growing areas and quantification of such growth, in particular, visualization and quantification systemically at the organ or tissue level (macroscopic level) have not been carried out [routinely].

When a therapeutic containing a radioactive nuclide that emits high-energy particles is administered to inactivate abnormally growing tissues or cells, it is necessary to pay close attention to the radioactive dose accumulated in vivo. The ICRP (International Committee for Radiation Protection) has prescribed permissible dosage levels for bone and the digestive tract. The systemic exposure levels have been determined at the national level. A conventional practice has been to count the dose for each tissue using a radiation instrument and to estimate whether the exposure dose in vivo is equal to or below the permissible dose level. However, these counting procedures do not allow visual identification or quantification of the in vivo dose distribution of the radioactive nuclide.

The present invention relates to a rapid screening method for new drug candidates by providing an early visual indication of pharmacological effects [growth suppression or inhibition] in vivo of drugs administered to living organism harboring an excessive growth of tissues or cells, such as malignant tumors and the like, and to a rapid method for establishing an appropriate use of therapeutic drugs. It further relates to a method for judging the optimum dosage for high-energy particles; a method for determining the ratio of the moiety retaining chemical stability of a therapeutic drug; a method for determining the lethal effects of high-energy particles; a method for determining the efficacy of drugs with tissue-specific efficacy; and a method for establishing an applicable dose of ⁹⁰Y, and the like.

### Disclosure of the invention

Extensive studies by the present inventors led to the discovery that when "thymidine" is intravenously administered to rats, the thymidine administered is utilized only in the S phase of the cell cycle, and more precisely, within 3 minutes after the administration of the thymidine. This observation is based on the findings obtained using a "thymidine" with the "2" carbon position within its structure labeled with ¹⁴C and tracking the fate of the administered thymidine. It was discovered that thymidine not incorporated during the cell division is rapidly metabolized in the liver, leucocytes, and the like and mostly excreted out of the body as carbon dioxide gas. Based on these findings, the present inventors discovered that a thymidine with "2" carbon position labeled with ¹⁴C is an extremely important marker for specifying the frequency of dividing cells in vivo and for localizing its site(s) within the body. It was also discovered that a (1,3-N) thymidine, obtained by attaching a fluorescent compound to the nitrogen atoms at 1,3-positions of the thymidine, can also be used as the labeled thymidine.

The present invention, based upon the above discovery, provides a method of identifying and quantifying abnormally growing versus normally growing tissues or cells by a two-dimensional image analysis of abnormally growing tissues or cells using [2-¹⁴C] thymidine or [1,3-N] fluorescent thymidine as the marker. The two-dimensional images can be visualized by macro-autoradiography.

The present invention provides a two-dimensional quantitative imaging method in which a radioactive nuclide which emits high-energy particles and [2- ¹⁴C] thymidine or [1,3-N] fluorescent thymidine are administered to a living organism; a biopsy specimen is prepared; the biopsy specimen is superimposed onto an x-ray photosensitive material, radiation absorber, and imaging plate (hereafter, sometimes abbreviated as I.P.); and photographic exposure or fluorescence analyzer instrument is used to obtain a simultaneous two-dimensional image of said radioactive nuclide and said thymidine, respectively. In this case, the high-energy particles that may be used are α-rays, -β electron beams, or heavy particles.

Further, the present invention, based the above two-dimensional quantitative imaging method, relates to a rapid method for screening a new drug candidate; to a rapid method for establishing an appropriate formula to use a therapeutic drug; further a method for determining the optimum dosage for high-energy particles; a method for determining the ratio of the moiety retaining chemical stability of a therapeutic drug; a method for determining the lethal effect of high-energy particles; a method for determining the efficacy of a drug exhibiting an tissue-specific efficacy; and a method for establishing an applicable dose for ⁹⁰Y, and the like.

First, this invention relates to a method which comprises administering in vivo a radioactive nuclide which emits high-energy particles, and then administering within a designated time, once or multiple times [2-¹⁴C] thymidine or [1,3-N] fluorescent thymidine; identifying and quantifying over time abnormally growing tissues or cells from normally growing-tissues or cells by a two-dimensional image analysis; thereby determining the optimum dose of the high-energy particles for inactivating the abnormally growing tissues or cells. In this case, the high-energy particles that may be used are α-rays, -β electron beams, or heavy particles.

Next, the invention relates to a method which comprises administering a therapeutic drug containing a radioactive nuclide that emits high-energy particles, and then administering [2-¹⁴C] thymidine or [1,3-N] fluorescent thymidine at a designated time once or multiple times; identifying and quantifying over time the abnormally growing tissues or cells and normally growing tissues or cells by a two-dimensional image analysis; thereby rapidly and accurately determining the ratio of the moiety retaining chemical stability and retention time of the therapeutic drug aimed at inactivating the abnormally growing tissue or cells.

Thirdly, the invention relates to a method which comprises administering in vivoa radioactive nuclide which emits high-energy particles, and then administering [2-¹⁴C] thymidine or [1,3-N] fluorescent thymidine at a designated time once or multiple times; identifying and quantifying over time the abnormally growing tissues or cells and normally growing tissues or cells by a two-dimensional image analysis; thereby rapidly and accurately determining the lethal effect of the high-energy particles on the abnormally growing tissues or cells.

Fourthly, the invention relates to a method which comprises administering in vivo a drug exhibiting an tissue-specific efficacy, and then administering [2-¹⁴C] thymidine or [1,3-N] fluorescent thymidine within a designated time before or after administration of the drug, once or multiple times; identifying and quantifying over time the abnormally growing tissues or cells and normally growing tissues or cells by a two-dimensional image analysis; thereby rapidly and accurately determining the efficacy of the drug exhibiting an tissue-specific efficacy.

Fifthly, the invention relates to a method for determining an applicable ⁹⁰Y dose which comprises administering in vivo ⁹⁰Y, and then administering [2-¹⁴C] thymidine or [1,3-N] fluorescent thymidine within a designated time once or multiple times; identifying and quantifying over time the abnormally growing tissues or cells and normally growing tissues or cells by a two-dimensional image analysis; thereby rapidly and accurately determining the dose for ⁹⁰Y applicable to suppress the growth of tumor at a specific site comprised of the abnormally growing tissue or cells and to reduce any associated pain.

Further, the present invention is a rapid screening method for new drugs which comprises screening new drug candidates by using the above two-dimensional quantitative imaging method, or a method derived therefrom, to determine the optimal dose of high-energy particles, to determine the ratio of the moiety retaining chemical stability of a therapeutic drug, to determine the lethal effect of high-energy particles, to determine the efficacy of a drug exhibiting an tissue-specific efficacy, and to establish an applicable dose for ⁹⁰Y, and the like.

It has been established above that based on this invention, it is possible to identify and quantify abnormally growing tissues or cells from normal tissues or cells by a two-dimensional quantitative imaging method, preferably by macro-autoradiography, based on the marker [2-¹⁴C] thymidine or [1,3-N] fluorescent thymidine; to provide simultaneous two-dimensional quantitative images for the radioactive nuclide and said thymidine, respectively; to make use of the above two-dimensional quantitative imaging method to determine the optimum dose of the high-energy particles, to determine the ratio of the moiety retaining chemical stability of a therapeutic drug, to determine the lethal effect of high-energy particles, to determine the efficacy of a drug exhibiting an tissue-specific efficacy, to establish an applicable dose for ⁹⁰Y, and to develop a rapid screening system for new drug candidates.

The procedures described above target not only tissues or cells in vivo, but can also target tissues or cells ex vivo by using the two-dimensional quantitative imaging method in the same manner, so that said two-dimensional quantitative imaging can be used to determine the optimal dose of high-energy particles, to determine the ratio of the moiety retaining the chemical stability of a therapeutic drug, to determine the lethal effects of high-energy particles, to determine the efficacy of drugs exhibiting an tissue-specific efficacy, to establish an applicable dose for ⁹⁰Y, and to screen for new drug candidates.

Specifically, similar results can be obtained by adding thereto a step for culturing the targeted tissue or cells from a living organism culturing ex vivo the targeted followed by similar analysis.

The present invention is a two-dimensional quantitative imaging method which comprises isolating tissue or cells from living organism, treating the isolated specimen with [2-¹⁴C] thymidine or [1,3-N] fluorescent thymidine, culturing the specimen, preparing sections and superposing the sections on radiation- or fluorescence-sensitive material, and carrying out a two-dimensional image analysis of the radiation or fluorescence of said thymidine, thereby quantifying the abnormally growing tissues or cells, from as normally grown tissues or cells. This two-dimensional image can be visualized by macro-autoradiography.

The two-dimensional quantitative imaging method permits the use of both a radioactive nuclide and labeled thymidine. That is, it comprises treating the tissue or cells isolated from a living organism with a radioactive nuclide that emits high-energy particles and [2-¹⁴C] thymidine or [1,3-N] fluorescent thymidine, culturing the specimen, preparing sections and superposing the sections on an x-ray photosensitive material, radiation absorber, and imaging plate, and exposing and using a fluorescence analyzer instrument to simultaneously obtain a two-dimensional image for said radioactive nuclide and said thymidine, respectively. In this case, the high-energy particles that may be used are α-rays, -β electron beams, or heavy particles.

The above two-dimensional quantitative imaging method can bring about a useful means for the prevention and treatment of diseases, particularly in the cancer treatment field. It is a method of determining the optimum dose for high-energy particles which comprises treating tissue or cells isolated from a living organism with a radioactive nuclide which emits high-energy particles, and then administering [2-¹⁴C] thymidine or [1,3-N] fluorescent thymidine at a designated time once or multiple times; culturing the specimen, identifying and quantifying over time the abnormally growing tissues or cells by said two-dimensional quantitative imaging method; thereby rapidly and accurately determining the optimum dose of the high-energy particles for inactivating the abnormally growing tissue or cells.

Next, the invention relates to a method which comprises treating the tissue or cells isolated from a living organism with a therapeutic drug containing a radioactive nuclide that emits high-energy particles, and then administering [2-¹⁴C] thymidine or [1,3-N] fluorescent thymidine at a designated time once or multiple times; identifying and quantifying over time the abnormally growing tissues or cells by said two-dimensional quantitative imaging method s; thereby rapidly and accurately determining the ratio of the moiety retaining chemical stability and retention time of the therapeutic drug aimed at inactivating the abnormally growing tissue or cells.

Thirdly, the invention relates to a method of determining the lethal effect, which comprises treating the tissue or cells isolated from a living organism with a radioactive nuclide which emits high-energy particles, and then administering [2-¹⁴C] thymidine or [1,3-N] fluorescent thymidine at a designated time once or multiple times; culturing the specimen, and identifying and quantifying over time the abnormally growing tissues or cells by said two-dimensional quantitative imaging method; thereby rapidly determining the lethal effect of the high-energy particles on the abnormally growing tissues or cells.

Fourthly, the invention relates to a method for determining the efficacy of a drug, which comprises treating the tissue or cells isolated from a living organism with a drug exhibiting an tissue-specific efficacy, and then administering [2-¹⁴C] thymidine or [1,3-N] fluorescent thymidine within a designated time before or after administration of the drug, once or multiple times; culturing the specimen, and identifying and quantifying over time the abnormally growing tissues or cells by said two-dimensional quantitative imaging method; thereby determining the efficacy of the drug exhibiting an tissue-specific efficacy.

Fifthly, the invention relates to a method for determining an applicable ⁹⁰Y dose which comprises treating the tissue or cells isolated from a living organism with ⁹⁰Y, treating the specimen within a designated time once or multiple times [2-¹⁴C] thymidine or [1,3-N] fluorescent thymidine; culturing the specimen; identifying and quantifying over time the abnormally growing tissues or cells by said two-dimensional quantitative imaging method; thereby determining the dose for ⁹⁰Y applicable to suppress the growth of tumor at a specific site comprised of the abnormally growing tissue or cells and to reduce any associated pain.

Further, the present invention is a screening system for new drug candidate, which comprises screening new drug candidates by using the above two-dimensional quantitative imaging method or methods derived therefrom, to determine the optimal dose of high-energy particles, to determine the ratio of the moiety retaining chemical stability of a therapeutic drug, to determine the lethal effect of high-energy particles, to determine the efficacy of a drug exhibiting an tissue-specific efficacy, and to establish an applicable dose for ⁹⁰Y, and the like.

### Brief Explanation of the Drawings

Figure 1 is a whole body autoradiograph obtained one hour after an intravenous administration of [2-¹⁴C] thymidine to a BALB/c (or Balb C) mouse on the seventh day after tumor implantation.
Figure 2 is a microautoradiograph of the digestive tract (small intestine) of the mouse shown in Figure 1. It shows basal intestinal gland cells.
Figure 3 is a microautoradiograph of the bone marrow (left lower part) and the cortex (right upper part) from a cross-section of the femur of the mouse shown in Figure 1.
Figure 4 is a microautoradiograph (T cells among the immune cells) of the spleen (mostly consisting of leucocytes and lymphoid cells).
Figure 5 is a microautoradiograph of growing cells at a abnormal growth site (Hu09).
Figure 6 is a whole body macroautoradiograph of a normal mouse obtained at 3 minutes after intravenous administration of [2-¹⁴C].
Figure 7 is a whole body autoradiograph of a normal BALB/c mouse at 10 minutes after intravenous administration of ⁹⁰Y.
Figure 8 is a whole body autoradiograph of a normal BALB/c mouse at 1 hour after intravenous administration of ⁹⁰Y.
Figure 9 is a whole body autoradiograph of a normal BALB/c mouse at 6 hours after intravenous administration of ⁹⁰Y.
Figure 10 is a whole body autoradiograph of a normal BALB/c mouse at 24 hours after intravenous administration of ⁹⁰Y.
Figure 11 is a whole body autoradiograph of a normal BALB/c mouse at 48 hours after intravenous administration of ⁹⁰Y.
Figure 12 is a microautoradiograph of the bone marrow showing the bone marrow cells of a mouse sacrificed one hour after administration of [2-¹⁴C] thymidine given at 48 hours following the administration of ⁹⁰Y.
Figure 13 is a microautoradiograph of the exposed mouse jejunum after treating with [2-¹⁴C] thymidine.
Figure 14 is a microautoradiograph of the isolated jejunum after treating with [2-¹⁴C] thymidine ⁹⁰Y and 48 hours later treated with [2-¹⁴C] thymidine.

### Best Embodiment for Carrying Out this Invention

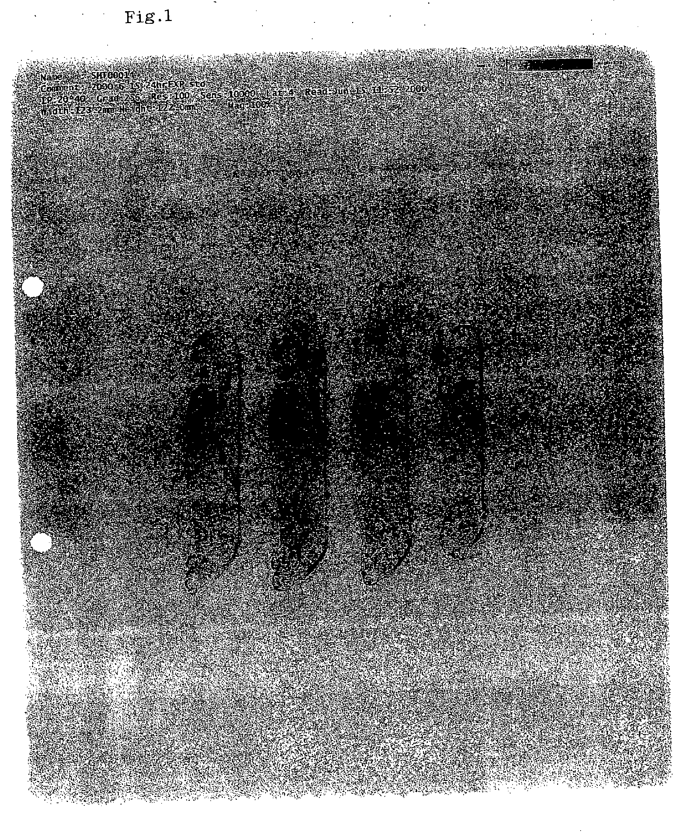

This invention is explained in detail based on preferred embodiment thereof as below:
The two-dimensional quantitative imaging method of this invention calls for analyzing a two-dimensional image of abnormally growing tissue or cells as marked by [2-¹⁴C] thymidine or [1,3-N] fluorescent thymidine (hereafter sometimes referred to as "labeled thymidine"), thereby identifying and quantifying from normally grown tissue or cells.
The two-dimensional image of this invention is preferably expressed by a macroautoradiograph [see Figure 1 etc], but is not limited, and can be achieved with a biopsy [tissue specimen obtained with a biopsy needle is allowed to float in a 1 mL culture solution, followed by adding said labeled thymidine, culturing the specimen, obtaining a thin sections after one hour of culture, and contact-exposing to a radioactive sensitive material to generate the two-dimensional image].
Instead of the photosensitive material used for the two-dimensional imaging by a macroautoradiograph, it is permissible in this invention to obtain the two-dimensional image by macroluminography using a FLA3000 model instrument, which records near ultraviolet light excited by red laser. Using such macroluminographic methods, one can intravenously inject said labeled thymidine, a marker for the physiological activity characteristics of the organ or tissue, particularly before and after an tissue-specific effective drug is administered in vivo, and use the whole body or a portion thereof as the specimen, thereby facilitating the determination of the pharmacological effect (efficacy) of said drug.
The two-dimensional quantitative imaging method of this invention is applicable to mammalian organisms such as nude mice, beagle dogs, miniature pigs, tumor-bearing rats, tumor-bearing mice, or other animal models of disease.

The abnormally growing tissue or cells, to which the two-dimensional quantitative imaging method of this invention is applicable, include tissues and cells of osteosarcoma, bone, liver cirrhosis, cell biopsy specimens of organs after transplantation, and in particular, the method is effective in the study of osteosarcoma and bones.

The present invention calls for identifying abnormally growing tissues or cells from normally growing tissues or cells by two-dimensional image analysis of [2-¹⁴C] or [1,3-N] as described above. In [2-¹⁴C] thymidine, the carbon at the position "2" is substituted by ¹⁴C; [1,3-N] fluorescent thymidine is one obtained by attaching a fluorescent compound to the nitrogen atom at the position 1 or 3 of the pyrimidine ring.

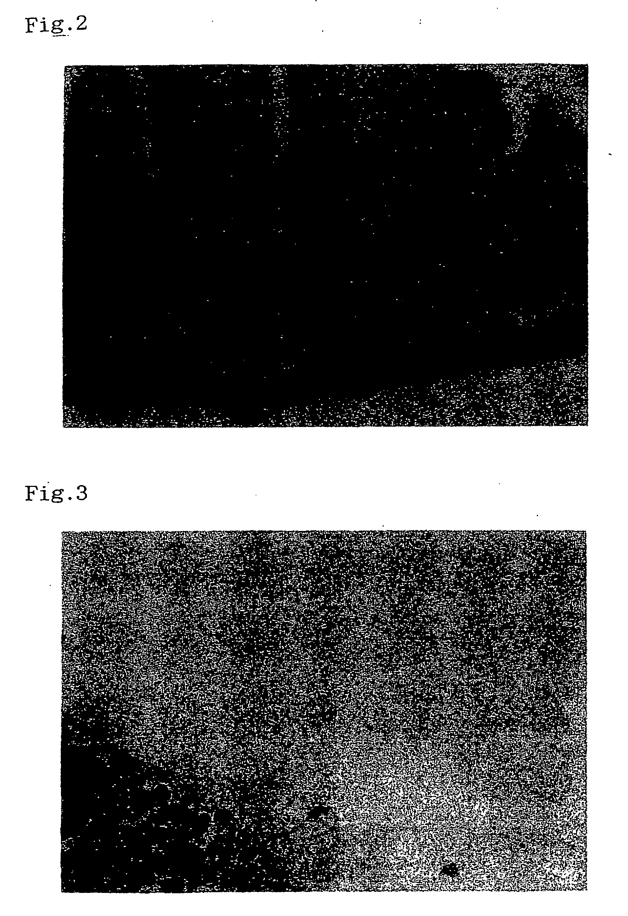

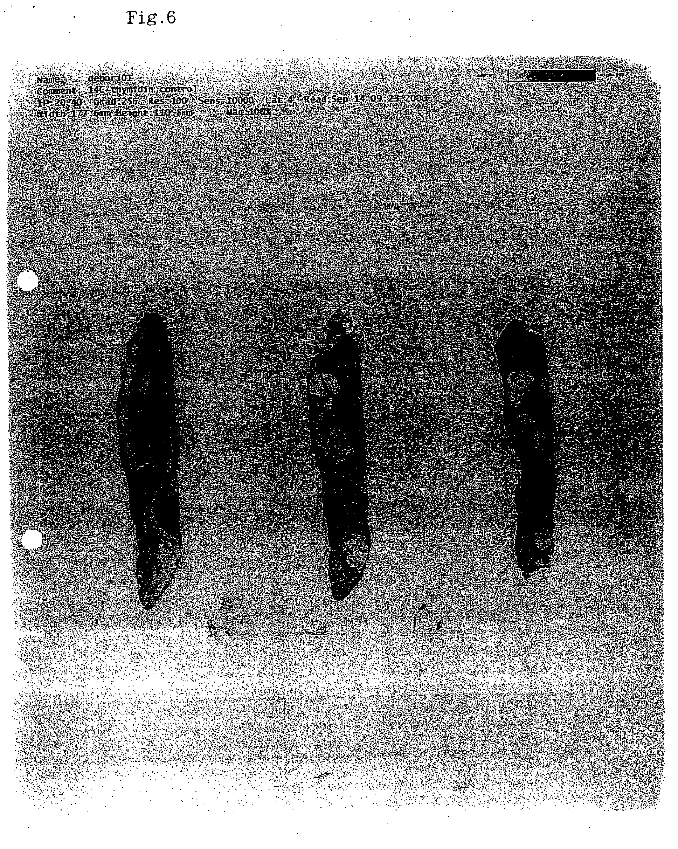

These labeled thymidine compounds function only for a short time (about three minutes) during DNA replication at the initial period of the cell division that occurs during the growth and regeneration of tissues. The sites where cell division is observed in normal cells include the basal layer of the digestive tract epithelium, splenic T cells, and young testis. [2-¹⁴C] is incorporated into the cell nucleus during the DNA synthesis in the S phase of the cell cycle in abnormally growing tissue or cells or is absorbed into the bone through the bone marrow leukocytes, so that a two-dimensional image analysis permits distinction among them. The ¹⁴C of [2-¹⁴C] emits radioactive β rays (half-life 5700 years). When the emitted energy hits the silver halide of the photographic emulsion (dry state) on the slice or section, elemental silver develops, which upon chemical processing gives silver grains (the small black dots in Figures 2 and 5). If the small black grains accumulate in the nucleus of a cell, the cell will divide into two cells after a day. On the other hand, if they are scattered only around the periphery of the nucleus, only cell degradation (lymphocytes and neutrophils) occurs, with no cell division.

[1,3-N] has a fluorescent compound bound to the nitrogen at position 1 or 3 of the pyrimidine ring in the chemical structure and is incorporated into the cell nucleus as in the case of [2-¹⁴C] during DNA synthesis; the unused portions of the compound are all excreted in the urine. Detection of the fluorescence of the [1,3-N] fluorescent thymidine incorporated allows the identification of the tissues or cells that have incorporated said [1,3-N].

Abnormally growing tissues or cells can be easily quantified according to this two-dimensional analysis by counting the number of nuclei with labeled thymidine incorporated into said cell nucleus per arbitrary area against other areas or merely measuring the intensity of the image per unit area (mm²).

The present invention calls for administering a radioactive nuclide that emits high-energy particles and [2-¹⁴C] thymidine or a fluorescent [1,3-N] thymidine to a living organism, preparing sections from a specimen obtained from the living organism, and superposing on it an x-ray photosensitive material, radiation absorber, and imaging plate for exposure or for fluorescence analysis, thereby obtaining simultaneously two-dimensional images of said radioactive nuclide and said thymidine.

With this method, one can determine the effect of administering a compound labeled by radioactive nuclide that emits high-energy particle. Such effects may be determined when the energy from the high-energy particles has sufficient effects on the targeted tissue or cells and the cell division is subsequently inhibited, while one can also confirm that if the radiation dose is insufficient, the inhibition of cell division is incomplete. That is, if the high-energy particles have adequate effects on the abnormal tissue or cell, the labeled thymidine is not detected, but if the radiation were not sufficiently effective, the inhibition is insufficient and labeled thymidine incorporation is detected in the abnormal tissue or cells.

The high-energy particles are α rays, -β electron beams, heavy particles, and the like. In particular, -β electron beams are preferably used for their concentrated cytotoxic effects only in the vicinity of the targeted abnormally growing tissue. The heavy particles are generally the so-called He rays, ionized particles of N, O, Ne and the like.

The radioactive nuclides that emit said high-energy particles include ³²p, ³³p, ⁹⁰Y, ⁸⁹Sr, and ¹⁶⁶Ho, and the like. For the preparation of sections of specimen from living organisms, the thickness is preferably 30-60 µm, more preferably about 40-60 µm.

The X-ray photosensitive materials include for example X-ray films, cold highly photosensitive materials (such as imaging plate), photostimulable luminants and the like. The radiation absorbers include for example aluminum foils, polyacrylic films, polyacrylic sheets, polyethylene, polyvinylidene chloride, Teflon™ films and the like. An imaging plate (IP) is a type of a cold light, high-sensitivity photosensitive material comprised of a substance which stores incident light or track energy of particles for a long period of time and then emits light when excited by an energy at a different wavelength (laser light and the like) (for example, a substance that stores radiation energy by conversion from Eu⁺² to Eu⁺³) . IP is a photostimulable luminant used to record images that can be digitized for computer analysis. The digital image is displayed and recorded per unit (unit: pixel) in an area 25µm x 25µm. Specifically, commercial IP products made by Fuji Film Co., Kodak Co., and Packard Co., can be used.

To said slice is superposed an X-ray photosensitive material, radiation absorber, or imaging plate, followed by exposing and then processing the image by chemical development or by acquiring the digital image by trapping the excited light generated with a red laser light. The fluorescence analytical instruments that may be used include FLA3000 and like.

The present invention, which calls for administering in vivo a radioactive nuclide that emits high-energy particles, then administering [2-¹⁴C] or [1,3-N] once or multiple times at a designated time, allows the distinction and quantification over time of abnormally growing tissues or cells compared to normal tissues or cells by a two-dimensional image analysis, thereby permitting the optimum dose of high-energy particles that inactivate the abnormally growing tissues or cells to be determined. High-energy particles that may be used are α-rays, -β electron beams, and heavy particles, preferably -β electron beams.

The in vivo dose of the radiation nuclide that emits high-energy particles should preferably be 3.7-370 kBq per 25g body weight. This corresponds to 8.8-880 MBq (240 µCi-24 mCi) per 60 kg body weight. The dosage of [2-¹⁴C] or [1,3-N] per administration should preferably be 3.7-370 kBq per 25 g body weight.

Abnormally growing tissues or cells are identified and quantified periodically at multiple time points and at designated times after the administration of the labeled thymidine given once or multiple times (hereafter applicable to the methods below). "Designated times" for the administration of the labeled thymidine should for example be 30 minutes, 1 hour, 6 hours, 24 hours, 3 days, 7 days, and the like (hereafter this will be applicable to all other methods below).

Results of the identification and quantification of the abnormally growing tissues or cells are compared against the known properties of dividing normal tissues or cells to divide, so as to confirm that the incorporation of the labeled thymidine into the abnormally growing tissues or into the cell nuclei is inhibited without harming the normal tissues or cells and to determine the optimum dose of the high-energy particles that eliminate abnormally growing tissues or cells.

In addition, the present invention provides a method which comprises administering in vivo a therapeutic drug containing a radioactive nuclide which emits high-energy particles, administering within a designated time, once or multiple times [2-¹⁴C] thymidine or [1,3-N] fluorescent thymidine; identifying and quantifying over time abnormally growing tissues or cells from normally growing tissues or cells by a two-dimensional image analysis; thereby rapidly and accurately determining the ratio of the moiety retaining chemical stability of the therapeutic drug, and its retention time, for inactivating the abnormally growing tissues or cells.

The quantity of the in vivo dose of a therapeutic drug containing a radioactive nuclide that emits high-energy particles should preferably be at 5 x 10⁻¹³ to 50 x 10⁻¹³ g per 25 g body weight. The dose per administration of [2-¹⁴C] or [1,3-N] is preferably 0.05 to 5.0 ng.

By identifying and determining over time the data from abnormally growing tissues or cells, and quantifying the ratio of migration to, and accumulation in, the normal tissues of a portions freed by metabolism from the parent compound bound to the high-energy particle ray nuclide, one can rapidly and accurately measure the ratio of the moiety retaining the chemical stability and retention time of a therapeutic drug used to inactivate the abnormally growing tissues or cells.

By the term "the ratio of the moiety retaining chemical stability of a therapeutic drug" is meant, for example, in the case of ⁹⁰Y bound to a monoclonal antibody, the moiety that binds the monoclonal antibody to ⁹⁰Y. Under conditions where ⁹⁰Y is not bound to a monoclonal antibody, ⁹⁰Y has no effect on the abnormally growing tissues or cells, so that the abnormally growing tissues or cells will continue to proliferate. This can be detected as incorporation of the labeled thymidine into the tissue or the cell and allows the rapidly and accurate determination of the ratio of the moiety retaining chemical stability and retention time of the therapeutic drug.

The present invention, which comprises administering a radioactive nuclide that emits high-energy particles, and administering [2-¹⁴C] thymidine or [1,3-N] fluorescent thymidine within a designated time, once or multiple times, identifies and quantifies abnormally growing tissues or cells over time by a two-dimensional image analysis, thereby enabling one to determine the lethal effect of the high-energy particles on the abnormally growing tissues or cells. The in vivo dose of the radiation nuclide that emits high-energy particles should preferably be 3.7-370 kBq per 25g body weight. The dosage of [2-¹⁴C] or [1,3-N] for one administration should preferably be 0.37-37 kBq per 25g body weight.

Based on the data from abnormally growing tissues or cells identified and quantified over time, confirmation that the incorporation of the labeled thymidine into the abnormally growing tissues or cell nuclei has been terminated enables one to determine rapidly the cytotoxic effects on the abnormally growing tissues or cells.

The present invention provides a method which comprises in vivo administration of a drug exhibiting tissue-specific efficacy, and the administering. [2-¹⁴C] thymidine or [1,3-N] fluorescent thymidine once or multiple times within a designated time before or after administration of the drug; identification and quantification of the abnormally growing tissues or cells over time by a two-dimensional image analysis; and thus the determination of the efficacy of the drug exhibiting the tissue-specific efficacy. Herein, the in vivo dose of drug should preferably be 0.05-5.0 pg per 25g body weight. Specific examples for the drugs are: ⁹⁰YCl, ⁹⁰YNO₃, ⁹⁰Y₂SO₄, ⁹⁰Y·CD20 antibody, ⁹⁰Y·CD11 antibody and the like. Per administration dose of [2-¹⁴C] thymidine or [1,3-N] fluorescent thymidine should preferably be 0.05-5.0 ng per 25g body weight.

The time period during which the toxic effect on the abnormally growing tissues or cells persists can be accurately determined by identifying and quantifying the abnormally growing tissues or cells over time. Further, the efficacy of a drug can be determined by checking the presence or absence of areas that resume cell division after said period has elapsed.

With the use of the two-dimensional quantitative imaging method of this invention in particular, a whole body image of ⁹⁰Y accumulation can be obtained at the organ or tissue level (macroscopic level). In particular, the ⁹⁰Y accumulation in osteosarcoma, bone marrow, and bones or distribution of portions of ⁹⁰Y in the liver and kidney can be confirmed as two-dimensional images.

The present invention provides a method for determining an appropriate ⁹⁰Y dose which comprises administering ⁹⁰Y in vivo, and administering [2-¹⁴C] thymidine or [1,3-N] fluorescent thymidine once or multiple times within a designated time; identifying and quantifying abnormally growing tissues or cells over time by a two-dimensional imaging method; and thereby determining the dose for ⁹⁰Y to be applied to suppress the growth of a specific tumor site containing abnormally growing tissues or cells and to reduce any associated pain.

The ⁹⁰Y used in this invention is the so-called carrier-free type (⁹⁰Y atoms only) and is completely different from the one used in recent years produced by the route ⁸⁹Y→⁹⁰Y (n→r).

Incidentally, ⁸⁹Sr approved as a radiopharmaceutical product in the United States and other countries is being used to alleviate pain in terminal osteosarcoma patients. The ⁸⁹Sr is prepared in a nuclear reactor by the n→r reaction of ⁸⁸Sr (a stable isotope) at yields as low as about 1/100 - 1/1000. For in vivo administration, ⁸⁸Sr that is present in large amounts in the ⁸⁹Sr is injected along with ⁸⁹Sr and is permanently deposited in the bone. This can affect the regenerative ability of the bone (osteoclastic and osteoblastic activity). ⁸⁹Sr is also a nuclide that emits β radiation at an energy level amounting to about 1.2 MeV and has a half-life of about 50 days, which is much longer than ⁹⁰Y (half-life 2.5 days), causing systemic radiation effects.

The radioactive nuclide ⁹⁰Y, which is preferred for this invention, is instead carrier-free as mentioned above, so that the deposition of elemental Y is negligible. The local sites of deposition of the elemental Sr in mammals is the bone and digestive tract, whereas ⁹⁰Y is not deposited and has no radiation effects on the digestive tract.

It is anticipated that the effect of ⁹⁰Y radiation energy on the bone marrow after the deposition of ⁹⁰Y in the bone increases with an increase in the administered dose of ⁹⁰Y. In this invention, a level at about 5 µCi/25 g showed cytotoxic effects near the sites of ⁹⁰Y deposition. However, since this can alleviate the pain caused by tumor cells present in the bone, an effective therapeutic strategy may be to perform transplantation of the bone marrow (or hepatocytes) after adequate cancer treatment.

The present invention permits visualization at an early stage of the pharmacological effects (growth suppression or inhibition) of a drug administered in vivo to living organisms harboring abnormally growing tissues or cells such as tumors. The two-dimensional imaging method of this invention requires about twenty-four hours for the imaging reaction and about three days for the production of the two-dimensional images, thereby enabling a rapid complete screening of new drug candidates.

The present invention uses [2-¹⁴C] thymidine or [1,3-N] thymidine which is only briefly functional (about three minutes) during the DNA replication step in the early period of the cell division that accompanies the growth and regeneration of tissue. This enables screening for anti-cancer drugs by the virtue of the differences in threshold values for suppressing cell division of normal and abnormal cells. That is, the present invention makes it possible to offer an anti-cancer drug having different threshold values for the suppression of normal and abnormal types of cell division.

Furthermore, the present invention can provide the present invention can provide a rapid screening system for new drugs characterized in that candidate of new drugs are screened wherein such method as the two-dimensional quantitative imaging method or methods derived therefrom is employed, to determine the above two-dimensional quantitative imaging method or methods derived therefrom, to determine the optimal dose of high-energy particles, to determine the ratio of the moiety retaining chemical stability of a therapeutic drug, to determine the toxic effects of high-energy particles, to determine the efficacy of drugs exhibiting an tissue-specific efficacy, and to establish an applicable dose for ⁹⁰Y, and the like. The embodiments explained above provide a rapid screening method for new drugs which comprises screening new drug candidates that target tissues or cells in vivo, as well as tissues or cells isolated from the organism by the application of the two-dimensional quantitative imaging method, thereby making use, based on said two-dimensional quantitative imaging method, of a method of determining the optimal dose of high-energy particles, a method of determining the ratio of the moiety retaining chemical stability of a therapeutic drug; a method for determining the toxic effects of high-energy particles; a method for determining the efficacy of a drug exhibiting an tissue-specific efficacy, and a method for establishing an applicable dose for ⁹⁰Y, and a method for screening a new drug candidate.

All embodiments that target in vivo tissue or cells can be applied unmodified to ex vivo applications using the tissue or cells isolated from the body, except that a step for culturing is added. Any further explanations omit embodiments targeted for tissue or cells isolated from the organism, but this omission no way limits the present invention. Embodiments that target tissue or cells isolated from the organism are very important and useful methods of prevention and treatment of diseases, and for clarifying specific uses of the prevention and treatment methods. For example, these can specify individually the appropriate dosage of an optimum therapeutic drug for a patient, thereby optimizing cancer prevention and treatment and greatly contributing to advancement in medical care.

### Examples

The present invention is now explained in detail by the following examples of this invention, but the invention is in no way limited to these examples.

### [Example 1]

The back of BALB/c nude mice was inoculated with a 10⁷ Hu09 cells (human osteosarcoma) grown in culture. 3 weeks later, animals with granulation tissue with a collagen layer were submitted for the experiment. A small portion, 2µCi (3.6 MBq), of 55 mCi/mmol (2GBq) [2-¹⁴C] thymidine (produced by Amersham-Pharmacia, Japan Co.) was injected into the tail vain, and one hour later the animal was sacrificed under ether-anesthesia and quickly frozen using liquid nitrogen. 50µm thick whole body sections cut longitudinally were prepared using a Leica Macrocut. The sections were stored in a sealed container and lyophilized. The dried sections in contact with a Fuji imaging plate were exposed in a dark box, and the IP was then processed with the Fuji BAS2000 analyzer to generate a digital image (Figure 1). Figure 1 shows the osteosarcoma on the back of the mouse as a dark image generated from a ¹⁴C soft electron beam image. ¹⁴C images were also detected in the digestive tract epithelium, bone marrow, bone, spleen, and skin, but no dark images were seen in the other major organs.

This shows that the except for the sites mentioned above, the [2-¹⁴C] thymidine is not utilized at all in the mouse in vivo. Analysis of a microautoradiograph of the darker silver grains indicated that the portions not participating in DNA replication were the bone and bone marrow. In the bone, there were no dividing cells immediately below the dark silver grains, while in the bone marrow the silver grains were located around the periphery of lymphoid cells (Figures 2, 3, 4, 5).

Figures 1-5 are now explained in detail. Figure 2 is a photograph (microautoradiograph) obtained as an enlargement of an autoradiograph by an optical microscope of the digestive tract (small intestine) of the mouse shown in Figure 1. Figure 2 shows that the labeled thymidine was incorporated (black spots) in the nuclei of newly generated epithelium and villous cells located at the base of the small intestinal mucosa.

Figure 3 is a microautoradiograph showing the bone marrow (left bottom section) and the cortex (right upper top) of a cross-section of femur of the mouse shown in Figure 1. Figure 3 shows that the surface of a large number of lymphoid cells in the bone marrow exhibit HCO₃ ions (small black spots) derived from the labeled thymidine, as well as black spots in the cortex.

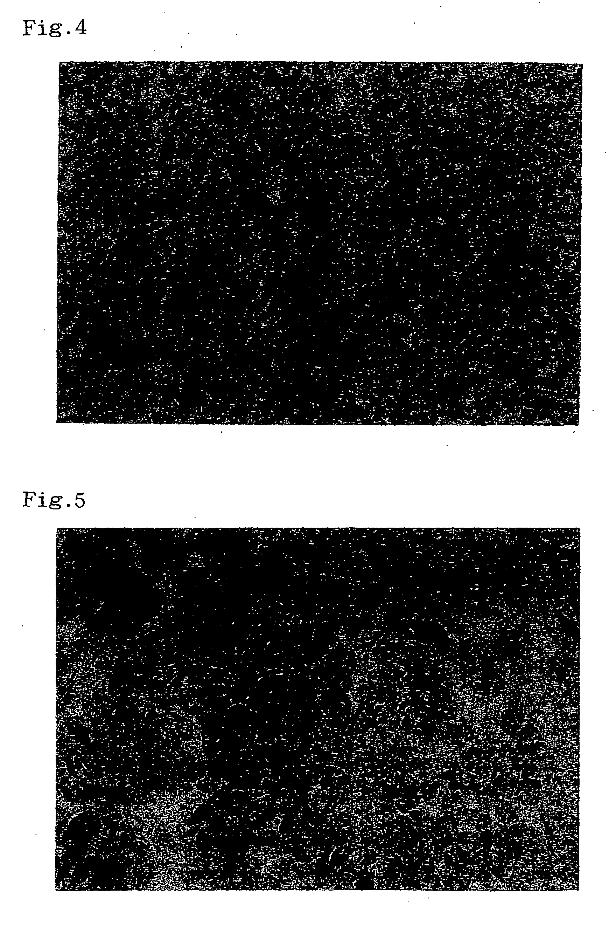

Figure 4 is a microradiograph (T lymphocytes) in the spleen showing normal mitosis during the lymphocyte growth. The T cells in Figure 4 (dark spots) differ from bone marrow cells in that they indicate the cells to be generated in the future and are present immediately above the cell nucleus.

Figure 5 is a microautoradiograph showing the cells present in areas of abnormal growth (HU90). The sites where there are black grains immediately above the cells in Figure 5 show the uptake of the labeled thymidine by cells destined for future cell proliferation.

A portion of tissues showing dark grains in the digestive tract mucosa, bone marrow, bone, spleen, skin, and osteosarcoma were placed in a sealed flask, which after treating with 2N sulfuric acid and heating under a nitrogen resulted in the generation of ¹⁴CO₂. Particularly greater amounts of ¹⁴CO₂ gas was generated by three tissues, bone marrow, bone, and spleen, especially the bone. The osteosarcoma also showed some ¹⁴CO₂ generation.

### [Example 2]

Figure 6 shows a whole body microautoradiograph of a normal mouse 3 minutes after intravenous administration of [2-¹⁴C] thymidine prepared under conditions similar to those of Example 1. Figure 6 shows a nearly uniform darkened image, except for the blackened image corresponding to the contents of the digestive tract. However, a detailed observation of the dark image of the ¹⁴C radiation indicates that the digestive tract mucosa, spleen, and skin exhibit darker images compared to other sites. This shows that [2-¹⁴C] thymidine is utilized during the DNA replication at these sites undergoing mitosis in the mouse during 3 minutes after the administration. It is evident from Figure 1 that the ¹⁴C component is completely excreted out of other areas in Figure 6 exhibiting the darkened images, except the bone and bone marrow, by 1 hour after the administration.

A normal mouse (BALB/c) was intravenously injected in the tail vein with an aqueous solution of [2-¹⁴C] thymidine at 2µCi/0.1ml, followed by freezing with liquid nitrogen and sectioning with a Leica Macrocut. The specimen was then placed in contact with IP, and 16 hours later the data was converted into a computer image for analysis. Analysis showed a nearly uniform distribution throughout the body. However, the figure (front middle) below Figure 6 of the intestinal mucosa indicates a high intensity in the bone (spine), while the upper and middle figures in Figure 6 show high intensities in the renal cortex and spleen.

### [Example 3]

Differences in effect of ⁹⁰Y high-energy particles (-β electron beams) on proliferating cells accompanying the growth of implanted human sarcoma HU09 cells and proliferating cells in normal tissues of a nude mouse were compared. ⁹⁰YCl₃ is a chloride of ⁹⁰Y available as a daughter nucleus from the mother nucleus of ⁹⁰Sr. Therefore, ⁹⁰Y does not contain any isotope element other than ⁸⁹y (so-called carrier-free). ⁹⁰Sr is generated by degradation of ²³⁵U, amounting to about 7% of the daughter nuclei from total degradation of ²³⁵U, but highly sophisticated technology is required to isolate pure ⁹⁰Sr . Furthermore, contamination with α particle-generating nuclides outgrown from ²³⁵U is not approved for use in radiopharmaceuticals in Japan.

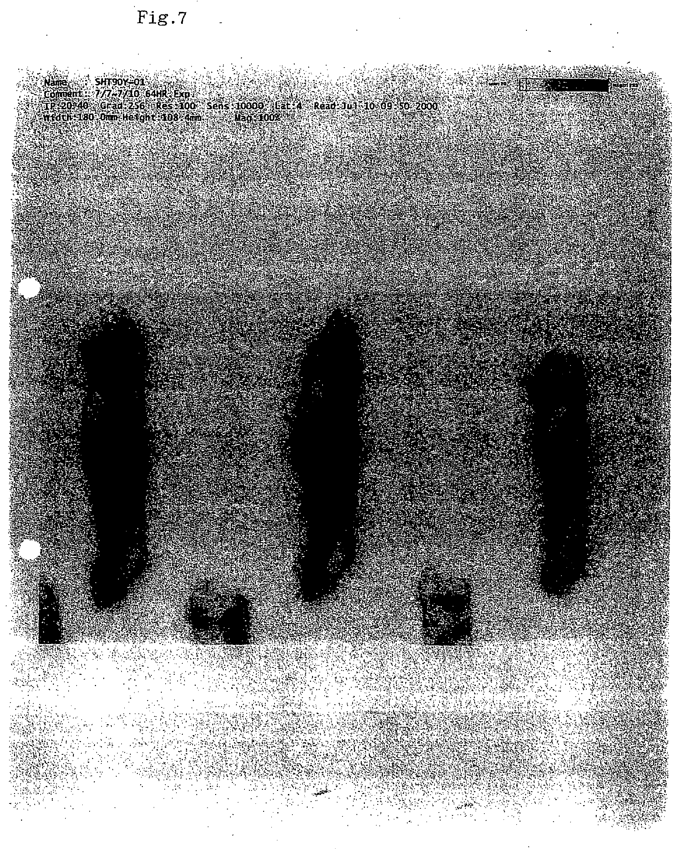

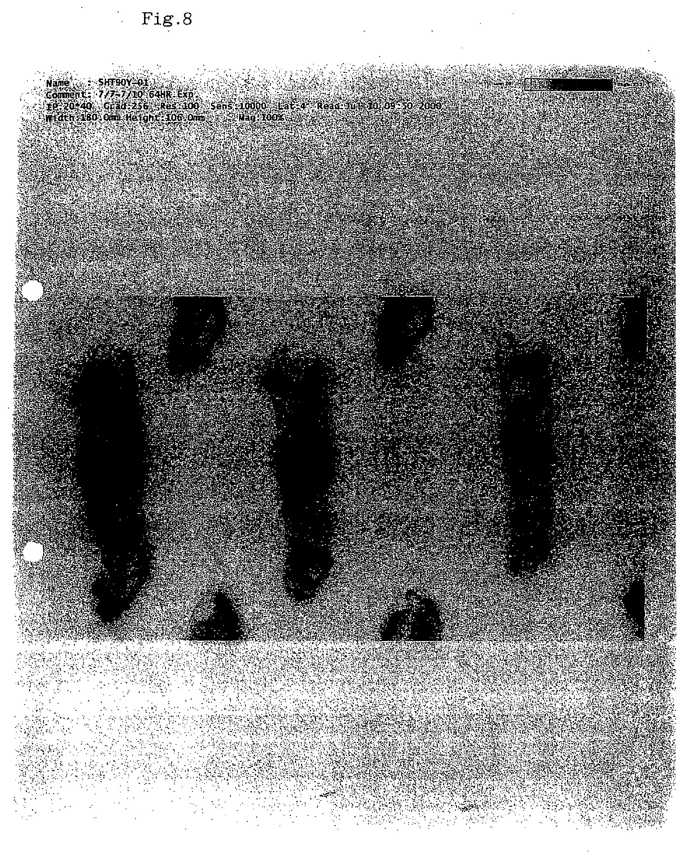

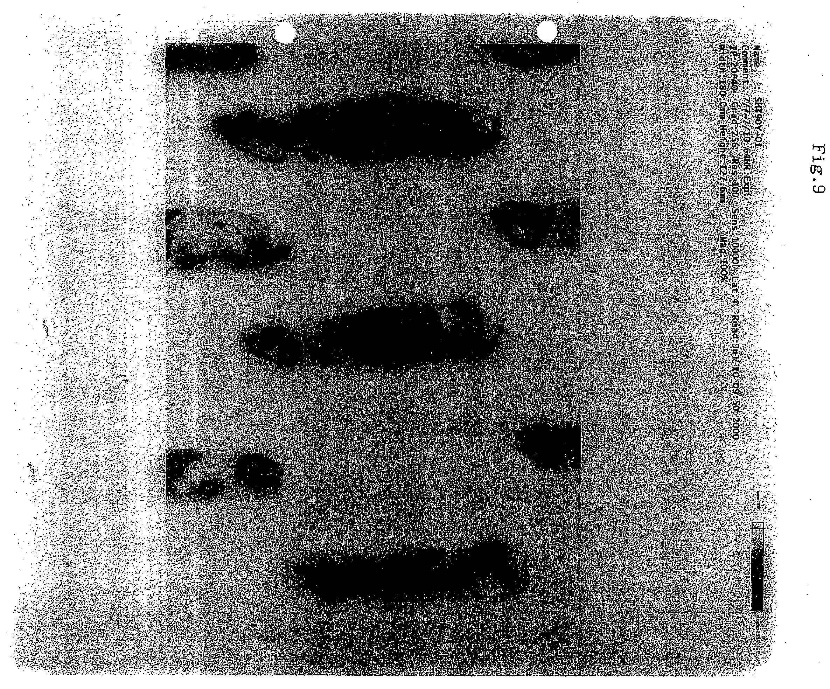

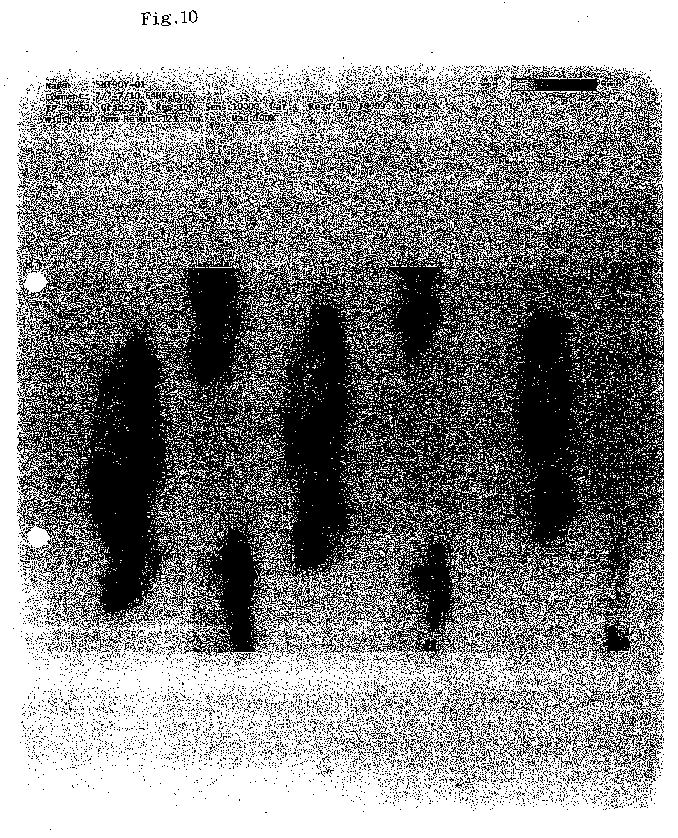

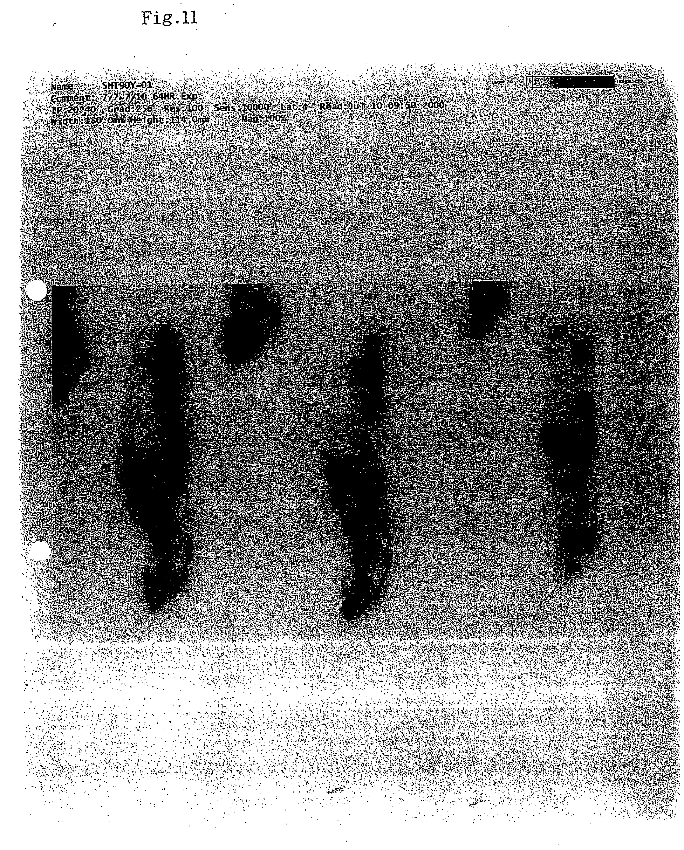

After administration of the aforementioned ⁹⁰Y into the mouse tail vein the systemic distribution was studied by the following experiments. The animals used were those obtained from the source as used for the BALB/c (body weight 25g) described in Examples 1 and 2 above. After administration of an aqueous 0.5µCi (18.5 kBq) ⁹⁰YCl citrate solution per male mouse in the tail vein, analysis was conducted over time at 5 time points: at 10 minutes, 1, 6, 24, and 48 hours. The animals were sacrificed under an ether anesthesia, and the whole body autoradiographs were prepared using with the same procedure as that used in Example 1 and Example 2 (Figures 7, 8, 9, 10, 11).

The IP images at each interval indicated that ⁹⁰Y localized in the mice with the skeletal bones showing the maximum concentration accumulation (70% or more of the total dose). The concentrations, in the other sites rapidly decreased over time after administration, with only a slight residue of ⁹⁰Y in the liver, spleen, and kidney in the animals at 24 hours after administration. The site of the osteosarcoma HU09 showed a blackened image completely equivalent to the extent seen in the bones of the mice. The analysis of these images show that ⁹⁰Y did not localize in the skin, digestive tract epithelium, and vascular endothelial cells that are considered to be undergoing constant tissue regeneration, but while localization was seen in the bone and bone marrow and a slight localization was seen in the liver and spleen. The results of Example 3 are extremely important and show that the energy of -β particles (electron beams) generated from ⁹⁰Y nuclide species amounts to 2.28 MeV, so that any living tissues within the range of about 20mm diameter from the site of concentration of this nuclide would be to some extent under conditions unsuitable for normal physiological activity. A sufficiently high ⁹⁰Y dose will lead to death. This dose is sharply proportional to abnormalities induced in neighboring cells and inversely proportionally to the square of the distance from the localized ⁹⁰Y site.

### [Example 4]

Since it was found that ⁹⁰Y is localized in the mouse body, the following experiments were conducted to elucidate the relationship between an increase in the radiation dose and the mitosis at the localized site.

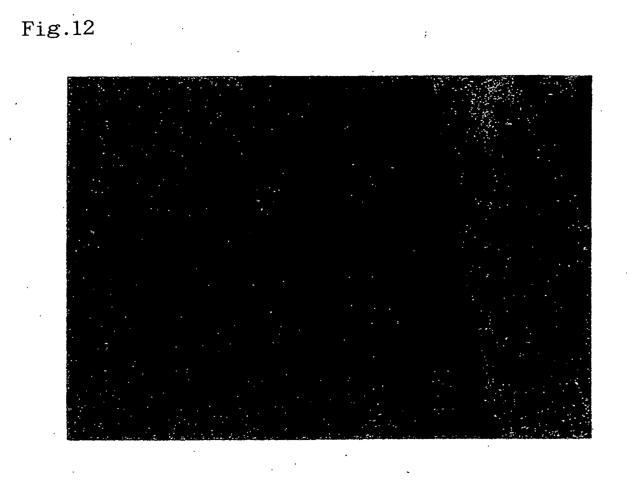

⁹⁰YCl at the three levels, 0.5 µCi (18.5kBq), 2 µCi (74 kBq) or 10 µCi (370 kBq), was injected intravenously into the tail of male BALB/c mice in groups of 6 mice per group (a total of 18 mice). [2-¹⁴C] thymidine at 2 µCi was administered to each ⁹⁰Y administered groups at 1, 6, 24, 48, 72 and 168 hours after administration. The animals were sacrificed one hour later under ether anesthesia, and the macroautoradio graphs were prepared as described in Example 1. In the first group after 0.5µCi administration, there was only a slight decrease in the activity of the lymphocytes in the bone marrow to degrade [2-¹⁴C] thymidine (Figure 12). Figure 12 is a microautoradiograph of the bone marrow, showing the bone marrow from a mouse sacrificed 1 hour after [2-¹⁴C] was administered, which was 48 hours after the ⁹⁰Y administration. As illustrated in Figure 12, there are essentially no dark silver grains observed in the bone and on the lymphoid cells in the bone marrow.

In the preparation of the image of Figure 12, the mouse was sacrificed one hour after the administration of a [2-¹⁴C] thymidine, and thin sections were prepared from the frozen mice. On the sections were placed five sheets of 10 µm thick aluminum foil, and this was brought into contact with IP to generate only the ⁹⁰Y image. This procedure does not give the [2-¹⁴C] thymidine image. After 6 half-lives (2.5 days x 6 = 15 days) of ⁹⁰Y or greater, the above whole body thin sections were exposed in direct contact with the IP to give the [2-¹⁴C] thymidine image. If the image of the two radiation nuclides is to be obtained simultaneously, one can place an industrial X-ray film in contact with said section, followed by five sheets of aluminum foil, and then the IP; these are then stored in a dark box. After about 7 days, the X-ray film is taken out and photographically processed by the usual method to produce the image, while the IP is processed by Fuji BAS2000 for computer processing of the images. This method provides ¹⁴C image on the industrial X-ray film. This method also gives a ⁹⁰Y image on the IP.

Table 1 shows the results of showing the effect of ⁹⁰Y radiation on the abnormally growing and normally growing cells obtained in this example. Table 1 shows the correlation, based on the [2-¹⁴C] thymidine incorporation as a marker of DNA replication, between the ability of the ⁹⁰Y radiation dose to affect abnormally growing tissues or cells and the normally growing tissues or cells versus the potential to suppress mitosis (marked tissues: digestive tract mucosa (m); bone marrow (B) tumor (T)).

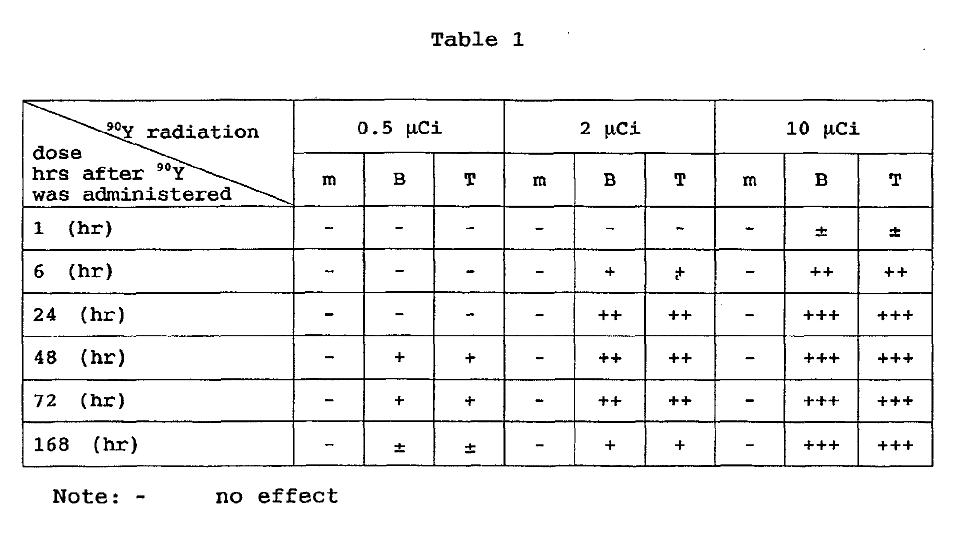

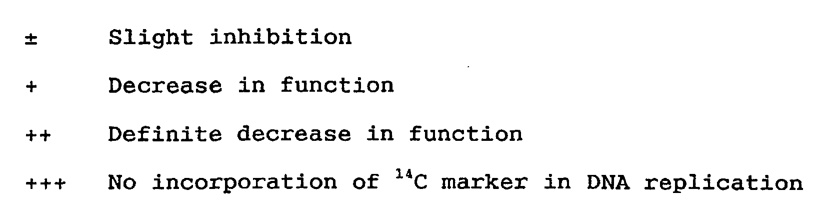

The results of the above example suggest the following. 1) [2-¹⁴C] thymidine is an excellent indicator for predicting mitosis about one day prior to DNA replication; 2) [2-¹⁴C] thymidine is incorporated into DNA in less than 3 minutes after injection into mammalian animals, and then rapidly loses its activity by metabolism, so that ¹⁴C is converted in vivo to ¹⁴CO₂, which is eliminated from the body; accordingly its use as a marker has no effects on the outcome; and 3) ¹⁴CO₂, is useful marker of bone formation reaction and may be used as a marker of bone formation within osteosarcoma and the highly differentiated fibroblastoma compact bone.

In addition, ⁹⁰Y emits extremely high-energy β rays at 2.28 MeV and affects living cells in proportion to its dosage. However, the range of the radiation is limited to a short distance and its maximum effective radius in vivo is about 10 mm. The ⁹⁰Y half-life is only about 2.5 days. Nevertheless, its handling requires much ingenuity to maintain safety. The present invention provides an excellent method for making use of ⁹⁰Y in a specific location in a living organism. The present invention now has opened a way to use ⁹⁰Y at an optimum dose to ensure a safe and maximum effect. An intravenous administration of ⁹⁰y, if physically and chemically highly pure, at a dose level of 2µCi/25g (equivalent to 5.6 mCi/70 kg, human body weight) to a living organism (including humans) is expected to have positive suppression of the abnormally growing osteosarcoma over 6 hours to 168 hours after the administration and to suppress insomnia, epidemic, headache, fever, loss of appetite, diarrhea, and the like that are predicted to accompany such growth. If the use of narcotics such as morphine can be reduced or terminated, it may be highly possible to utilize its efficacy as an anti-cancer therapeutic agent in conjunction with progress in cancer chemotherapy.

### [Example 5]

In a manner similar to that of Example 2, [2-¹⁴C] thymidine was intravenously injected to mice, and then a biopsy sections obtained 3 minutes after the injection was subjected to two-dimensional macroautoradiographic analysis. The macroautoradiograph showed characteristic autoradiographic features such as the incorporation of [2-¹⁴C] thymidine in the basal layer of the digestive mucosa in the small and large intestines. This establishes that even in a short time of about 3 minutes, [2-¹⁴C] thymidine is incorporated into cells destined to divide within a very short duration of the S1 phase (an early division phase in the cell cycle). Based on this data, experiments were conducted to study the incorporation of [2-¹⁴C] thymidine at the crypts of the small intestine in an in vitro experimental system (an experiment in which a specimen removed from a living organism is cultured), as shown in the following example.

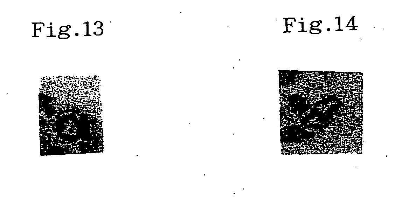

The specimen used was the jejunum from a healthy 7-week old Wistar rat, male. Under ethyl ether anesthesia, the abdomen was opened to expose the jejunum, which was marked with methylene blue. Near the mark, 0.05 ml of an aqueous [2-¹⁴C] thymidine solution with 5µCi (185 kBq) was injected into the supporting tissue at the boundary between the muscular and mucosal layers and left standing 5 minutes; the marked section was ligated at a 2 cm length and was fixed frozen using liquid nitrogen. Frozen sections were prepared with a microtome, and a semi-microautoradiographs were obtained. Figure 13 shows the result. The darkened images were clearly recorded in the basal layer of the intestinal mucosa.

Next, the jejunum of a healthy Wistar male rat at seven weeks of age was used. The part of the abdomen was opened under ethyl ether anesthesia, and the jejunum of about 2-4 cm in length was excised. An aqueous [2-¹⁴C] thymidine solution at 5µCi (185 kBq) in 0.05 ml was injected into the supporting tissue at the boundary of muscular and mucous layers of the excised jejunum by the method described above, and then the specimen was immediately immersed in a Waymouth culture procedure (containing protamine 12 x 10%) and cultured for 10 minutes at 37°C. Then, the specimen was immediately frozen in liquid nitrogen and sectioned using a microtome to obtain frozen sections. Semi-microautoradiographs were prepared from these sections.

Figure 14 clearly shows the substantially darkened images present in the basal layer of the intestinal tract mucosa. The observed incorporation of [2-¹⁴C] thymidine makes it clear that the presence of mitotic activity can be determined satisfactorily even in an in vitro experimental system.

These experimental results show that even in a simple test tube (in vitro) system, [2-¹⁴C] thymidine can be used in methods to evaluate the ability of a cell to divide (when the reagent is incorporated into a cell nucleus, mitosis reliably occurs within a period of 1-2 days, so that the ratio of the number of cells proven to have incorporated and the number of cells with no incorporation can be quantitatively expressed as the mitotic activity of a group of cells (or tissue)) in a sensitive manner and that a simple in vitro test result can quantitatively estimate an in vivo assay using a complex live organism, so that the practical value of this method is extremely high.

### Potential for Industrial Applications

The present invention provides a method for visualizing at an early period the pharmacological effects [growth suppression or inhibition] of drugs administered to living organisms harboring abnormally growing tissues or cells, such as a malignant tumor, rapidly completing the screening of new drug candidates, and establishing an appropriate use of a therapeutic drug. It further provides a method for determining the optimum dosage of high-energy particles; a method for determining the ratio of the moiety retaining chemical stability of a therapeutic drug; a method for determining the toxic effects of high-energy particles; a method for determining the efficacy of a drug exhibiting an tissue-specific efficacy; and a method for establishing the optimum dose for ⁹⁰Y, and the like.

These provide extremely useful procedures in cancer prevention and cancer treatment, thereby substantially contributing to advances in the cancer treatment.

## Claims

1. A two-dimensional quantitative imaging method comprising distinguishing and quantifying abnormally growing tissues and cells from normally growing tissue or cells in a living organism by a two-dimensional image analysis based on the marker [2-¹⁴C] thymidine or [1,3-N] fluorescent thymidine.

2. A two-dimensional quantitative imaging method as set forth in Claim 1, wherein said two-dimensional image is expressed by a macroautoradiograph.

3. A two-dimensional quantitative imaging method which comprises administering a radioactive nuclide which emits high-energy particles and [2- ¹⁴C] thymidine or [1,3-N] fluorescent thymidine to a living organism; preparing a biopsy specimen; superposing on the biopsy specimen an X-ray photosensitive material, radiation absorber, and imaging plate; and then developing the film or analyzing the image using a fluorescence analyzer instrument, thereby simultaneously obtaining a two-dimensional image for said radioactive nuclide and said thymidine, respectively.

4. A two-dimensional quantitative imaging method as set forth in Claim 3 wherein the high-energy particles are α-rays, -β electron beams, or heavy particles.

5. A method for determining the optimum dose of high-energy particles which comprises administering in vivo a radioactive nuclide which emits high-energy particles; administering [2-¹⁴C] thymidine or [1,3-N] fluorescent thymidine within a designated time once or multiple times; distinguishing and quantifying the abnormally growing tissues or cells from normal grown tissues or cells over time by the two-dimensional quantitative imaging method as set for the in any one of the Claims 1 to 4, thereby allowing the determination of the optimum dose of the high-energy particles for inactivating the abnormally growing tissues or cells.

6. A method for determining the optimum dose as set forth in Claim 5, wherein said high-energy particles are α rays, -β electron beams, or heavy particles.

7. A method for determining the ratio of the moiety retaining chemical stability and retention time of a therapeutic drug, which comprises administering in vivo a radioactive nuclide that emits high-energy particles; administering [2-¹⁴C] thymidine or [1,3-N] fluorescent thymidine at a designated time once or multiple times; and identifying and quantifying over time the abnormally growing tissues or cells by the two-dimensional quantitative imaging method as set forth in any one of the claims 1 to 4; thereby rapidly and accurately determining the ratio of the moiety retaining chemical stability and retention time of the therapeutic drug aimed at inactivating the abnormally growing tissue or cells.

8. A method for determining the lethal effects, which comprises administering in vivo a radioactive nuclide which emits high-energy particles; administering [2-¹⁴C] thymidine or [1,3-N] fluorescent thymidine at a designated time once or multiple times; and identifying and quantifying abnormally growing tissues or cells over time by the two-dimensional quantitative imaging method as set forth in any one of the claims 1 to 4; thereby rapidly determining the lethal effect of the high-energy particles on the abnormally growing tissues or cells.

9. A method for determining the efficacy, which comprises administering in vivo a drug exhibiting an tissue-specific efficacy; administering [2-¹⁴C] thymidine or a [1,3-N] fluorescent thymidine once or multiple times within a designated time before or after administration of the drug; and identifying and quantifying abnormally growing tissues or cells over time by the two-dimensional quantitative imaging method as set forth in any one of the claims 1 to 4; thereby determining the efficacy of the drug exhibiting an tissue-specificity.

10. A method for determining an applicable dose for ⁹⁰Y, which comprises administering in vivo ⁹⁰Y; administering [2-¹⁴C] thymidine or a [1,3-N] fluorescent thymidine within a designated time once or multiple times; and identifying and quantifying abnormally growing tissues or cells over time by the two-dimensional quantitative imaging method as set forth in any one of the claims 1 to 4; thereby determining the dose for ⁹⁰Y to be applied to suppress the growth of a tumor at a specific site comprised of the abnormally growing tissue or cells and to reduce any associated pain.

11. A screening system for new drug candidates, which comprises screening new drug candidates using the two-dimensional quantitative imaging method as set forth in any one of the claims 1 to 4 or the method as set forth in any one of the claims 5 to 10.

12. A two-dimensional quantitative imaging method, which comprises isolating a portion of tissue or cells from a living organism; administering [2-¹⁴C] thymidine or [1,3-N] fluorescent thymidine to said isolated portion ex vivo; culturing; preparing a section therefrom; superposing a radiation or fluorescence photosensitive material to be in contact with said section; and distinguishing and quantifying the abnormally growing tissues or cells from normally growing tissues or cells by a two-dimensional image analysis of the radiation or fluorescence of said thymidine.

13. A two-dimensional quantitative imaging method as set forth in Claim 12, wherein said two-dimensional image is visualized by macroautoradiography.

14. A two-dimensional quantitative imaging method, which comprises administering to isolated tissue or cells a radioactive nuclide which emits high-energy particles and [2-¹⁴C] thymidine or [1,3-N] fluorescent thymidine ex vivo; culturing; preparing a section from said tissue or cells; superimposing thereonto an X-ray photosensitive material, radiation absorber, and imaging plate; and exposing or measuring with a fluorescence analyzer instrument, thereby acquiring simultaneously two-dimensional images of said radioactive nuclide and said thymidine, respectively.

15. A two-dimensional quantitative imaging method as set forth in claim 15, wherein the high-energy particles are α-rays, -β electron beams, or heavy particles.

16. A method for determining the optimum dose of high-energy particles, which comprises administering a radioactive nuclide which emits high-energy particles ex vivo to a tissue or cells isolated from a living organism; administering [2-¹⁴C] thymidine or [1,3-N] fluorescent thymidine at a designated time once or multiple times; and identifying and quantifying abnormally growing tissues or cells over time by the two-dimensional quantitative imaging method as set forth in any one of the claims 12 to 15; thereby determining the optimum dose of the high-energy particles for inactivating the abnormally growing tissues or cells.

17. A method for determining the optimum dose as set forth in claim 16, wherein the high-energy particles are α-rays, -β electron beams, or heavy particles.

18. A method for determining the ratio of the moiety retaining chemical stability and retention time of a therapeutic drug, which comprises administering a radioactive nuclide which emits high-energy particles to a tissue or cells isolated ex vivo from a living organism; administering [2-¹⁴C] thymidine or [1,3-N] fluorescent thymidine at a designated time once or multiple times; and identifying and quantifying abnormally growing tissues or cells over time by the two-dimensional quantitative imaging method as set forth in any one of the claims 12 to 15; thereby determining the ratio of the moiety retaining chemical stability and retention time of the therapeutic drug aimed at inactivating the abnormally growing tissue or cells.

19. A method for determining a lethal effect, which comprises administering ex vivo a radioactive nuclide which emits high-energy particles to a tissue or cells isolated from a living organism; then administering [2-¹⁴C] thymidine or [1,3-N] fluorescent thymidine at a designated time once or multiple times; and identifying and quantifying abnormally growing tissues or cells over time by the two-dimensional quantitative imaging method as set forth in any one of the claims 12 to 15; thereby rapidly determining the lethal effect of the high-energy particles on the abnormally growing tissues or cells.

20. A method for determining drug efficacy, which comprises administering ex vivo a drug exhibiting an tissue-specific efficacy to a tissue or cells isolated from a living organism; administering [2-¹⁴C] thymidine or a [1,3-N] fluorescent thymidine once or multiple times within a designated time before or after the administration of said drug; culturing; and identifying and quantifying over time the abnormally growing tissues or cells by the two-dimensional quantitative imaging method, as set forth in Claims 12 to 15; thereby determining the efficacy of the drug exhibiting tissue-specificity.

21. A method for determining an applicable dose for ⁹⁰Y, which comprises administering ⁹⁰Y ex vivo to a tissue or cells isolated from a living organism; administering [2-¹⁴C] thymidine or [1,3-N] fluorescent thymidine at a designated time once or multiple times; culturing; and identifying and quantifying abnormally growing tissues or cells over time by the two-dimensional quantitative imaging method as set forth in any one of the claims 12 to 15, thereby determining the dose for ⁹⁰Y to be applied to suppress the growth of a tumor at a specific site comprised of the abnormally growing tissue or cells and to reduce any associated pain.

22. A screening system for new drug candidates, which comprises screening new drug candidates using the two-dimensional quantitative imaging method as set forth in any one of the claims 12 to 15 or the method as set forth in any one of the claims 16 to 21.
